# EUROPEAN PATENT APPLICATION

(11) **EP 4 491 093 A1**
(43) Date of publication of application: **15.01.2025**
(21) Application number: 22930773.1
(22) Date of filing: 08.03.2022
(51) Int. Cl.: A61B 1/00, A61B 1/24

(54) **ASSISTANCE TOOL, IMAGING DEVICE, PROGRAM, AND METHOD**

(71) Applicant: Aillis Inc., Tokyo, 1040028 (JP)
(72) Inventor: SAKUMA Akiho, Tokyo 104-0028 (JP); YASUMI Takashi, Tokyo 104-0028 (JP); TAKAHASHI Wataru, Tokyo 104-0028 (JP); HASEGAWA Fuminari, Kobe-shi, Hyogo 650-0027 (JP)
(74) Representative: Reddie & Grose LLP
(86) International application number: PCT/JP2022/010014
(87) International publication number: WO 2023/170788

(57) **Abstract**

An assistance tool that is suitable for imaging an oral cavity of a subject person and assists in the imaging, and an imaging device, a program, and a method for imaging an inner side of the oral cavity using the assistance tool are provided.

Provided is an assistance tool to be inserted into an oral cavity for imaging an inner side of the oral cavity by an imaging device, the assistance tool including: a main body having a shape; an opening being formed at an end portion of the main body in a direction opposite to a direction where the assistance tool is inserted into the oral cavity in a manner that the imaging device can be inserted into an interior of the main body; a cover being formed at an end portion of the main body in the direction where the assistance tool is inserted into the oral cavity in a manner that light detected by the imaging device can pass through the cover; and a cover collar being formed to connect the main body and the cover in a manner that the cover is disposed at the end portion of the main body in the direction where the assistance tool is inserted into the oral cavity, at least a part of the cover collar being included within an angle of view imaged by the imaging device inserted from the opening.

## Description

### Technical Field

The present disclosure relates to an assistance tool that is suitable for imaging an oral cavity of a subject person and assists in the imaging, and an imaging device, a program, and a method for imaging an inner side of the oral cavity using the assistance tool.

### Background Art

Conventionally, it has been known that doctors diagnose, for example, a viral cold or the like by observing a change in a state of an oral cavity of a subject person. Non Patent Literature 1 reports that there is a specific pattern for influenza in lymphatic follicles appearing at a deepest part of a pharynx located inside an oral cavity. The lymphatic follicles having this specific pattern are called influenza follicles, which are a characteristic sign of influenza, and are said to appear about 2 hours after onset. However, such pharynx portion has been diagnosed by the doctors through direct visual inspection, and diagnosis using images has not been made.

### Citation List

### Non Patent Literature

Non Patent Literature 1: Miyamoto and Watanabe, "Posterior Pharyngeal Wall Follicles as a Diagnostic Marker of Influenza During Physical Examination: Considering Their Meaning and Value" Journals of Nihon University Medical Association 72(1):11-18 (2013)

### Summary of Invention

### Technical Problem

Therefore, based on the technology as described above, an object of the present disclosure is to provide an assistance tool that is suitable for imaging an oral cavity of a subject person and assists in the imaging, and an imaging device, a program, and a method for imaging an inner side of the oral cavity using the assistance tool, according to various embodiments.

### Solution to Problem

According to one aspect of the present disclosure, provided is "an assistance tool to be inserted into an oral cavity for imaging an inner side of the oral cavity by an imaging device, the assistance tool including: a main body having a shape; an opening being formed at an end portion of the main body in a direction opposite to a direction where the assistance tool is inserted into the oral cavity in a manner that the imaging device can be inserted into an interior of the main body; a cover being formed at an end portion of the main body in the direction where the assistance tool is inserted into the oral cavity in a manner that light detected by the imaging device can pass through the cover; and a cover collar being formed to connect the main body and the cover in a manner that the cover is disposed at the end portion of the main body in the direction where the assistance tool is inserted into the oral cavity, and in a manner that at least a part of the cover collar is reflected within an angle of view imaged by the imaging device inserted from the opening".

According to one aspect of the present disclosure, provided is "an imaging device to be inserted into an interior of a main body of an assistance tool from an opening of the assistance tool, the assistance tool being inserted into an oral cavity for imaging an inner side of the oral cavity, and including: the main body in a cylindrical shape; the opening being formed at an end portion of the main body in a direction opposite to a direction where the assistance tool is inserted into the oral cavity; a cover being formed at an end portion of the main body in the direction where the assistance tool is inserted into the oral cavity; and a cover collar being formed to connect the main body and the cover in a manner that the cover is disposed at the end portion of the main body in the direction where the assistance tool is inserted into the oral cavity, the imaging device including: at least one processor, wherein the at least one processor is configured to perform a process of starting capturing a subject image by receiving an operation input by a user, and based on the captured subject image, and outputting information indicating that the imaging device is not inserted into the assistance tool in a case where at least a part of the cover collar is not included as a result of determining whether or not at least the part of the cover collar is included in the subject image".

According to one aspect of the present disclosure, provided is "a program that causes an imaging device to be inserted into an interior of a main body of an assistance tool from an opening of the assistance tool to function as a processor configured to perform a process of starting capturing a subject image by receiving an operation input by a user, and based on the captured subject image, outputting information indicating that the imaging device is not inserted into an assistance tool in a case where at least a part of a cover collar is not included as a result of determining whether or not at least the part of the cover collar is included in the subject image, wherein the assistance tool is inserted into an oral cavity for imaging an inner side of the oral cavity, and includes: the main body in a cylindrical shape; the opening being formed at an end portion of the main body in a direction opposite to a direction where the assistance tool is inserted into the oral cavity; a cover being formed at an end portion of the main body in the direction where the assistance tool is inserted into the oral cavity; and the cover collar being formed to connect the main body and the cover in a manner that the cover is disposed at the end portion of the main body in the direction where the assistance tool is inserted into the oral cavity".

According to one aspect of the present disclosure, provided is "a method that is executed by at least one processor included in an imaging device to be inserted into an interior of a main body of an assistance tool from an opening of the assistance tool, the assistance tool being inserted into an oral cavity for imaging an inner side of the oral cavity, and including: the main body in a cylindrical shape; the opening being formed at an end portion of the main body in a direction opposite to a direction where the assistance tool is inserted into the oral cavity; a cover being formed at an end portion of the main body in the direction where the assistance tool is inserted into the oral cavity; and the cover collar being formed to connect the main body and the cover so that the cover is disposed at the end portion of the main body in the direction where the assistance tool is inserted into the oral cavity, the method including: a stage of starting capturing a subject image by receiving an operation input by a user, and a stage of outputting information indicating that the imaging device is not inserted into the assistance tool in a case where at least a part of the cover collar is not included as a result of determining whether or not at least the part of the cover collar is included in the subject image based on the captured subject image".

### Advantageous Effects of Invention

According to the present disclosure, it is possible to provide the assistance tool that is suitable for imaging the oral cavity of the subject person and assists in the imaging, and the imaging device, the program, and the method for imaging the inner side of the oral cavity using the assistance tool.

Note that the above effects are merely exemplary for convenience of description, and are not restrictive. In addition to or instead of the above effects, any effect described in the present disclosure or any effect obvious to a person skilled in the art can also be exhibited.

### Brief Description of Drawings

Fig. 1 is a view illustrating a use state of a processing system 1 according to one embodiment of the present disclosure.
Fig. 2 is a view illustrating a use state of the processing system 1 according to one embodiment of the present disclosure.
Fig. 3A is a schematic view of the processing system 1 according to one embodiment of the present disclosure.
Fig. 3B is a top view illustrating a configuration of an imaging device 200 according to one embodiment of the present disclosure.
Fig. 4 is a perspective view illustrating a configuration of an assistance tool 300 according to one embodiment of the present disclosure.
Fig. 5 is a side view illustrating a configuration of the assistance tool 300 according to one embodiment of the present disclosure.
Fig. 6 is a schematic view illustrating use states of the imaging device 200 and the assistance tool 300 according to one embodiment of the present disclosure.
Fig. 7 is a schematic view illustrating how the imaging device 200 and the assistance tool 300 are attached according to one embodiment of the present disclosure.
Fig. 8A is a schematic view illustrating a configuration of the assistance tool 300 in a front view according to one embodiment of the present disclosure.
Fig. 8B is a schematic view illustrating a configuration of a part of a distal end 314 of the assistance tool 300 in a cross-sectional view according to one embodiment of the present disclosure.
Fig. 9 is a schematic view illustrating the configuration of the assistance tool 300 in the front view according to one embodiment of the present disclosure.
Fig. 10 is a schematic view illustrating a configuration of the imaging device 200 in a cross-sectional view according to one embodiment of the present disclosure.
Fig. 11 is a schematic view illustrating a configuration of the assistance tool 300 in a top view according to one embodiment of the present disclosure.
Fig. 12 is a schematic view illustrating a configuration of a positioning mechanism 329 of the assistance tool 300 according to one embodiment of the present disclosure.
Fig. 13 is a schematic view illustrating a configuration of a plate 334 of the assistance tool 300 according to one embodiment of the present disclosure.
Fig. 14 is a block diagram illustrating a configuration of the imaging device 200 according to one embodiment of the present disclosure.
Fig. 15 is a chart illustrating a processing flow executed in the imaging device 200 according to one embodiment of the present disclosure.
Fig. 16 is a chart illustrating a processing flow regarding generation of a learned model according to one embodiment of the present disclosure.
Fig. 17 is a view illustrating an example of a screen output in the imaging device 200 according to one embodiment of the present disclosure.
Fig. 18 is a view illustrating an example of a screen output in the imaging device 200 according to one embodiment of the present disclosure.

### Description of Embodiments

Various embodiments of the present disclosure will be described with reference to the accompanying drawings. Note that common components in the drawings are denoted by the same reference numerals.

### First Embodiment

### 1. Overview of Processing System 1

The processing system 1 according to the present disclosure is mainly used to obtain a subject image in which an inner portion of an oral cavity of a subject person is imaged. In particular, the processing system 1 is used to image a back of a throat area of the oral cavity, specifically, a pharynx. Accordingly, in the following description, a case where the processing system 1 according to the present disclosure is used for imaging the pharynx will be mainly described. However, the pharynx is merely an example of a site to be imaged, and as a matter of course, the processing system 1 according to the present disclosure can be suitably used for other sites such as tonsils in the oral cavity.

The processing system 1 according to the present disclosure is used to determine a possibility of contracting a predetermined disease from a subject image obtained by imaging a subject including at least a pharyngeal area of the oral cavity of the subject person, and to diagnose or assist the diagnosis for the predetermined disease. An example of the disease determined by the processing system 1 is influenza. Usually, the possibility of contracting the influenza is diagnosed by examining the pharynx or a tonsil area of the subject person or determining presence or absence of findings such as follicles in the pharyngeal area. However, it is possible to perform the diagnosis or the assistance of the diagnosis by determining the possibility of contracting the influenza using the processing system 1 and outputting a result of the determination. Note that the determination of the possibility of contracting the influenza is an example. The processing system 1 can be suitably used to determine any disease in which differences appear in findings in the oral cavity due to the contracting. Note that the difference in the findings are not limited to those found by a doctor or the like, and medically known to exist. For example, a difference that can be recognized by a person other than a doctor or a difference that can be detected by artificial intelligence or image recognition technology can be suitably applied to the processing system 1. Examples of such disease include, in addition to the influenza, a hemolytic streptococcal infection, an adenovirus infection, an EB virus infection, a mycoplasma infection, infections such as a hand, foot and mouth disease, herpangina and candidiasis, diseases exhibiting vascular disorders or mucosal disorders such as arteriosclerosis, diabetes and hypertension, and tumors such as oral cancer, tongue cancer and pharyngeal cancer.

Note that, in the present disclosure, terms such as "determination" and "diagnosis" for a disease are used, but these terms do not necessarily mean a definite determination or diagnosis by a doctor. For example, it is also possible to naturally include determination and diagnosis with a processing device 100 included in the processing system 1 with the processing system 1 of the present disclosure used by the subject person himself/herself or used by a user other than a doctor.

Further, in the present disclosure, the subject person to be imaged by the imaging device 200 can include any human such as a patient, an examinee, a diagnostic user, and a healthy person. Further, in the present disclosure, a user who holds the imaging device 200 and performs imaging operation is not limited to a medical worker such as a doctor, a nurse, or a laboratory technician, and can include any human such as the user himself/herself. The processing system 1 according to the present disclosure is typically assumed to be used in a medical institution. However, the present disclosure is not limited to this case, and the place for using the processing system may be any place such as the user's home, school, or workplace.

Further, in the present disclosure, each of the subject person and the user is merely used as a term for distinguishing each of the humans, and the subject person and the user may be different from each other or may be the same. In other words, in a case where a person captures an image of an inner side of his/her oral cavity using the imaging device, the person can be both the subject person and the user.

Further, in the present disclosure, as described above, the subject may include at least a part of the oral cavity of the subject person. Further, the disease to be determined may be any disease in which differences appear in the findings in the oral cavity. However, in the following description, a case will be described in which the subject includes the pharynx or a pharyngeal area and the possibility of contracting the influenza as the disease is determined.

Further, in the present disclosure, the subject image or a determination image may be one or a plurality of moving images or one or a plurality of still images. As examples of operations, if a power button is pressed down, a through image is fetched by a camera, and the captured through image is displayed on a display 203. Thereafter, if a capture button is pressed down by the user, the one or the plurality of still images are captured by the camera, and the captured image is displayed on the display 203. Alternatively, if the capture button is pressed down by the user, capture of a moving image is started, and the image being captured by the camera during that period is displayed on the display 203. Then, if the capture button is pressed down again, the capture of the moving image ends. In this way, in a series of the operations, various images such as the through image, the still image, and the moving image are captured by the camera, and displayed on the display. Moreover, there may be a processed image obtained by subjecting the subject images or the determination images to various image processes for the display and the process according to the present disclosure. However, the subject images and the determination images do not refer to only a specific image among these images, but can include all of these images captured by the camera. Further, such subject images do not all need to include at least a part of the oral cavity, and as described above, there is also a case where at least the part of the oral cavity is not included in the through image or the like.

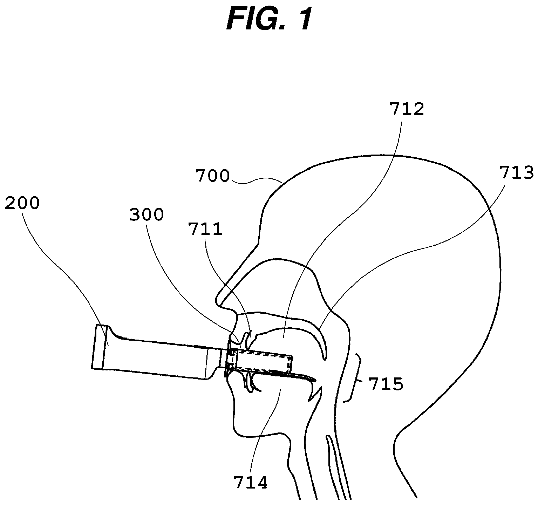

Fig. 1 is the view illustrating the use state of the processing system 1 according to one embodiment of the present disclosure. According to Fig. 1, the processing system 1 according to the present disclosure includes the processing device 100 and the imaging device 200. The user attaches an assistance tool 300 in a manner that a distal end of the imaging device 200 is covered with the assistance tool, and inserts the imaging device 200 into an oral cavity 710 of the subject person together with the assistance tool 300. Specifically, first, the user (who may be a subject person 700 himself/herself or may be one different from the subject person 700) attaches the assistance tool 300 in the manner that the distal end of the imaging device 200 is covered with the assistance tool 300. Then, the user inserts the imaging device 200 to which the assistance tool 300 is attached into the oral cavity 710. At this time, the distal end of the assistance tool 300 passes through an incisor 711 and is inserted to an area near a soft palate 713. In other words, the imaging device 200 is similarly inserted to the area near the soft palate 713. At this time, a tongue 714 is pushed downward by the assistance tool 300 (functioning as a tongue depressor), and movement of the tongue 714 is restricted. Accordingly, it is possible for the user to secure a good field of view of the imaging device 200 and perform excellent imaging of a pharynx 715 located in front of the imaging device 200. Further, in the imaging by the imaging device 200, the imaging device 200 is inserted into an interior of the oral cavity 710 of the subject person. Therefore, by covering the distal end of the imaging device 200 with the assistance tool 300, it is possible to prevent a mucous membrane, saliva, a body fluid, or the like in the oral cavity from directly coming into contact with the imaging device 200, and prevent contamination of the imaging device 200. Note that, in a case where such assistance tool 300 is not used, at least a distal end portion of the imaging device 200 needs to be subjected to a process such as disinfection and cleaning each time of use. Accordingly, it is desirable to use the assistance tool 300 also from a viewpoint of saving the user's trouble.

The captured subject image (typically, an image including the pharynx 715) is transmitted from the imaging device 200 to the processing device 100 communicably connected via a wired or wireless network. The processor of the processing device 100 that has received the subject image processes a program stored in a memory, to select the determination image to be used for the determination from the subject images, and determine the possibility of contracting the predetermined disease using the determination image. Then, a result is output to the display or the like.

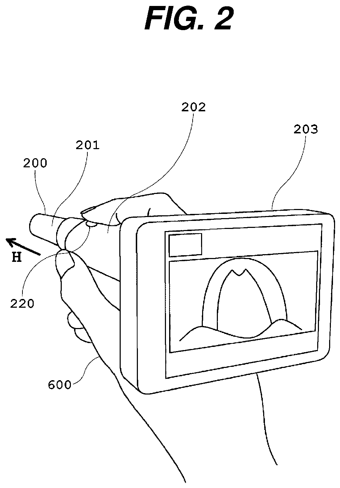

Fig. 2 is the view illustrating the use state of the processing system 1 according to one embodiment of the present disclosure. Specifically, Fig. 2 is a view illustrating a state where the imaging device 200 of the processing system 1 is gripped by a user 600. According to Fig. 2, the imaging device 200 includes a main body 201, a grip 202, and the display 203 from a side to be inserted into the oral cavity. The main body 201 and the grip 202 are formed in a substantially columnar shape having a predetermined length along an insertion direction H into the oral cavity. Further, the display 203 is disposed on a side of the grip 202 opposite to the main body 201 side. Therefore, the imaging device 200 is entirely formed in a substantially columnar shape, and held by the user 600 in a holding manner as holding a pencil. In other words, since a display panel of the display 203 faces a direction of the user 600 in the use state, it is possible to easily handle the imaging device 200 while checking the subject image captured by the imaging device 200 in real time.

Further, if the user 600 holds the grip 202 in a direction where the subject image is displayed in a normal direction on the display 203, a capture button 220 is configured to be disposed on an upper surface side of the grip. Therefore, when the user 600 holds the grip, the user 600 can easily press down the capture button 220 with an index finger or the like.

### 2. Configuration of Processing System 1

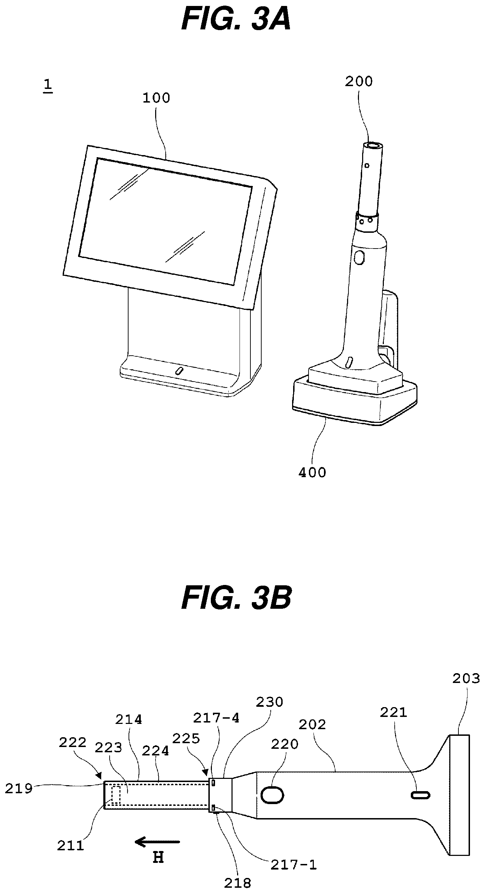

Fig. 3 is the schematic view of the processing system 1 according to one embodiment of the present disclosure. According to Fig. 3, the processing system 1 includes the processing device 100 and the imaging device 200 communicably connected to the processing device 100 via the wired or wireless network. The processing device 100 receives an operation input by the user, and controls the imaging by the imaging device 200. Further, the processing device 100 processes the subject image captured by the imaging device 200 to determine the possibility of the subject person contracting the influenza. Moreover, the processing device 100 outputs a result determined, and notifies the subject person, the user, the doctor, or the like of the result.

The distal end of the imaging device 200 is inserted into the oral cavity of the subject person to image the inner side of the oral cavity, particularly the pharynx. A specific process thereof will be described later. The captured subject image is transmitted to the processing device 100 via the wired or wireless network.

Note that the processing system 1 can further include a mounting table 400 as necessary. The imaging device 200 can be stably mounted on the mounting table 400. Further, the mounting table 400 is connected to a power source via a wired cable, whereby power can be supplied from a power supply terminal of the mounting table 400 to the imaging device 200 through a power supply port of the imaging device 200.

### 3. Configuration of Imaging Device 200

The processing system 1 according to the present disclosure includes the imaging device 200 that captures an image to be processed by the processing device 100. Fig. 3B is the top view illustrating the configuration of the imaging device 200 according to one embodiment of the present disclosure. Hereinafter, a specific configuration of the imaging device 200 will be described with reference to Fig. 3B.

According to Fig. 3B, the main body 201 includes a proximal end 225 and a distal end 222, and is formed in a columnar body having a predetermined length in a direction where light is emitted from a light source 212, that is, in a direction substantially parallel to the insertion direction H in the oral cavity. Then, at least the distal end 222 of the main body 201 is inserted into the oral cavity.

The main body 201 is formed in a columnar shape as a hollow cylindrical shape with a cross section being a perfect circle. A wall portion 224 may be made of any material as long as the material can guide the light into the wall portion, and as an example, the wall portion can be obtained using a thermoplastic resin. As the thermoplastic resin, polyolefin-based resins such as a chain polyolefin-based resin (a polypropylene-based resin or the like) and a cyclic polyolefin-based resin (a norbornene-based resin or the like), cellulose ester-based resins such as triacetyl cellulose and diacetyl cellulose, polyester-based resins, polycarbonate-based resins, (meth) acrylic resins, polystyrene-based resins, or mixtures and copolymers thereof are used. In other words, the wall portion 224 of the main body 201 functions as a light guide body for guiding the light emitted from the light source into the oral cavity or toward a direction of a diffusion plate.

Since the main body 201 is formed in a hollow shape, an accommodation space 223 is formed therein with the wall portion 224. A camera 211 is accommodated in the accommodation space 223. Note that the main body 201 only needs to be formed in a columnar shape with the accommodation space 223. Accordingly, the accommodation space 223 does not need to have a cylindrical shape with a cross section being a perfect circle, and may have an elliptical or polygonal cross section. Further, an inner portion of the main body 201 does not necessarily need to be formed hollow.

A distal end of the grip 202 is connected to the proximal end 225 of the main body 201. The user holds the grip 202, and performs operations such as insertion and removal of the imaging device 200. The grip 202 is formed in a columnar body having a predetermined length in the direction substantially parallel to the insertion direction H in the oral cavity, that is, along a longitudinal direction of the main body 201, and disposed on the same straight line as the main body 201 in the direction H. Note that, in the present embodiment, a cross section of the grip 202 in a vertical direction is formed to be substantially elliptical, but the cross section is not necessarily elliptical, and may be a perfect circle, an ellipse, or a polygon.

In the grip 202, a connecting portion 230 is formed at a position closest to the proximal end 225 of the main body 201, and the grip 202 is connected to the main body 201 with the connecting portion 230 therebetween. Engagement protrusions 217 (217-1 to 217-4) for positioning the assistance tool 300 and a positioning protrusion 218 are formed on an outer periphery of the connecting portion 230. The engagement protrusions 217 engage with engagement protrusions 318 (318-1 to 318-4) formed on the assistance tool 300. Further, the positioning protrusion 218 is inserted into an insertion hole 330 formed in the assistance tool 300 to position the imaging device 200 and the assistance tool 300 with respect to each other. Note that, in the present embodiment, as the engagement protrusions 217 of the main body 201, four engagement protrusions (the engagement protrusions 217-1 to 217-4) in total are arranged at equal intervals on a surface of the grip 202 at a position close to the proximal end 225 of the main body 201. Further, one positioning protrusion 218 is disposed between the engagement protrusions 217 on the surface of the grip 202 at the position close to the proximal end 225 of the main body 201. However, the present disclosure is not limited thereto, and it is also possible to dispose only one of the engagement protrusions 217 and the positioning protrusion 218. Further, the number of the engagement protrusions 217 and the positioning protrusion 218 may be one or more, and may be any number.

Further, the grip 202 includes the capture button 220 at a position close to the proximal end 225 of the main body 201 on the upper surface of the grip, that is, near the distal end of the grip 202 in the insertion direction H in the oral cavity. In this way, when the user 600 holds the grip, the user 600 can easily press down the capture button 220 with the index finger or the like. Further, a power button 221 is disposed on the upper surface of the grip 202 at a position close to the display 203, that is, at a position opposite to the capture button 220 of the grip 202. In this way, it is possible to prevent the power button 221 from being erroneously pressed down when the user 600 holds the grip and performs the imaging.

The display 203 has a substantially rectangular parallelepiped shape as a whole, and is disposed on the same straight line as the main body 201 in the direction H. Further, the display 203 includes a display panel on a surface in a direction (that is, a direction toward the user) opposite to the insertion direction H in the oral cavity. Accordingly, the display 203 is formed in a manner that a surface including the display panel is substantially perpendicular to the longitudinal direction of the main body 201 and the grip 202 formed to be substantially parallel to the insertion direction H in the oral cavity. Then, the display is connected to the grip 202 on a side opposite to the oral cavity of the grip 202 on a surface facing the surface including the display panel. Note that a shape of the display is not limited to the substantially rectangular parallelepiped shape, and may be any shape such as a cylindrical shape.

Here, the imaging device 200 can be mounted in a perpendicular direction so that the surface including the display panel is in contact with a mounting surface. Therefore, at least an outer periphery of the surface including the display panel of the display 203 is formed on a substantially plane suitable for the mounting.

A diffusion plate 219 is disposed at the distal end 222 of the main body 201, and diffuses the light emitted from the light source 212 and passing through the main body 201 toward the inner side of the oral cavity. The diffusion plate 219 has a shape corresponding to a cross-sectional shape of a portion of the main body 201 capable of guiding the light. In the present embodiment, the main body 201 is formed in a hollow cylindrical shape. Accordingly, a cross section of the diffusion plate 219 is also formed in a hollow shape corresponding to that shape.

The camera 211 is used to generate the subject image by detecting reflected light diffused from the diffusion plate 219, emitted into the oral cavity, and reflected on the subject. The camera 211 is disposed on the same straight line as the main body 201 in the direction H inside the wall portion 224 of the main body 201, that is, in the accommodation space 223 formed inside the main body 201. Note that although only one camera 211 is described in the present embodiment, the imaging device 200 may include a plurality of cameras. By generating the subject image using the plurality of cameras, the subject image includes information related to a three-dimensional shape. Further, in the present embodiment, the camera 211 is disposed in the accommodation space 223 of the main body 201, but the camera 211 may be disposed at the distal end 222 of the main body 201 or on the main body 201 (may be inside the main body 201 or on an outer periphery of the main body 201).

### 4. Configuration of Assistance Tool 300

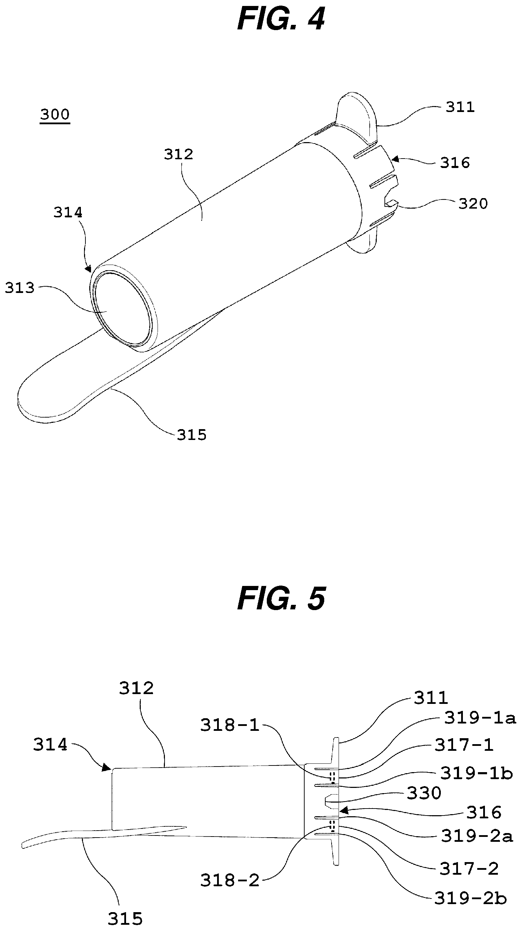

Fig. 4 is the perspective view illustrating the configuration of the assistance tool 300 according to one embodiment of the present disclosure. The assistance tool 300 is a tool for assisting in imaging the inner side of the oral cavity of the subject person. Accordingly, in a case where the oral cavity is imaged by the imaging device 200, the assistance tool is not always necessary, and may not be used as desired by the user or the subject person. At least a part of the imaging device 200 on the distal end side is inserted into the assistance tool 300. Accordingly, at least a part of the assistance tool 300 preferably has translucency from a viewpoint of securing a good field of view without interfering with the imaging device 200.

In the present embodiment, the assistance tool 300 is manufactured by integral molding of a resin. However, the assistance tool may be manufactured by other materials such as paper, cloth, and metal, or a combination thereof. Further, the assistance tool 300 is desirably a disposable type, but may be a reusable type. Examples of such resin include the thermoplastic resin. As the thermoplastic resin, the polyolefin-based resins such as the chain polyolefin-based resin (the polypropylene-based resin or the like) and the cyclic polyolefin-based resin (the norbornene-based resin or the like), the cellulose ester-based resins such as the triacetyl cellulose and the diacetyl cellulose, the polyester-based resins, the polycarbonate-based resins, the (meth) acrylic resins, the polystyrene-based resins, vinyl-based resins, nylon-based resins, polyurethane, fluorine-based resins or mixtures and copolymers thereof are used. Note that, in the present embodiment, it is preferable to configure the assistance tool 300 that is easily deformed according to selection of a material or a thickness of the material during processing. With such configuration, it is possible to easily dispose the assistance tool 300, for example, in a case where the assistance tool is the disposable type as described above.

According to Fig. 4, the assistance tool 300 includes a main body 312 formed in a cylindrical shape, a pair of gripping plates 311 disposed at a proximal end 316 of the main body 312, and a tongue depressor 315 disposed near the distal end 314 of the main body 312.

Since the main body 312 is formed to cover at least a part of the main body 201 on the distal end 222 side of the imaging device 200 inside thereof, the main body is formed in a cylindrical shape having a predetermined length along the direction H that is the direction where the assistance tool 300 is inserted into the oral cavity, corresponding to the length of the main body 201. Then, an opening 320 for inserting the imaging device 200 is disposed on the proximal end 316 side, and a cover 313 for passing irradiation light from the light source 212 of the imaging device 200 and reflected light from the subject is disposed on the distal end 314 side. Note that, in the present embodiment, the assistance tool 300 is inserted into the oral cavity from the distal end 314.

Further, in the present embodiment, the main body 201 of the imaging device 200 has a substantially constant inner diameter and outer diameter from the proximal end 225 to the distal end 222. Accordingly, the main body 312 of the assistance tool 300 is also correspondingly shaped so that the inner diameter and the outer diameter are substantially constant along the longitudinal direction. Note that a cross-sectional shape of the main body 312 is preferably formed corresponding to a cross-sectional shape of the main body 201 of the imaging device 200, but may be any shape as long as the main body 201 can be inserted, and may be any shape such as a perfect circle, an ellipse, or a polygon.

The pair of gripping plates 311 is formed along the proximal end 316 of the main body 312, to extend toward an outer side of the main body 312 in the direction perpendicular or substantially perpendicular to a direction (that is, the direction H) where the assistance tool 300 is inserted into the oral cavity. In other words, with this configuration, the gripping plates 311 are used to be held by a hand when the user inserts the assistance tool 300 into the oral cavity of the subject person. In the present embodiment, when the assistance tool 300 is inserted into the oral cavity, surfaces on distal end sides of the gripping plates 311 are in contact with lips of the subject person or the like, and thus the gripping plates also function as a restricting member for restricting further insertion of the assistance tool 300 and is capable of preventing aspiration. Further, since the gripping plates 311 are formed to extend outward, it is possible to prevent the assistance tool 300 itself from rolling on a floor surface or the like. Note that, in the present embodiment, the pair of gripping plates 311 is disposed on the proximal end 316 side of the main body 312 to be symmetric in an up-and-down direction, but may be formed in a doughnut shape along the proximal end 316 of the main body 312, and may have any shape.

The tongue depressor 315 is disposed at a lower portion of the main body 312 (a side in a lingual direction in the oral cavity), and is formed on a blade toward the lingual direction. During the imaging with the imaging device 200, the tongue of the subject person moves in front of the imaging device 200, whereby the imaging in the oral cavity is inhibited. The tongue depressor 315 pushes the tongue downward to restrict movement of the tongue in the oral cavity and prevent the tongue from being located in front of the imaging device 200. Accordingly, in the present embodiment, the tongue depressor 315 is formed in a blade shape, but may have any shape as long as this function can be implemented.

Fig. 5 is a side illustrating an overview of a configuration of the assistance tool 300 in a side view according to one embodiment of the present disclosure. According to Fig. 5, as described above, the assistance tool 300 includes the tongue depressor 315 near the distal end 314 side of the main body 312 and the gripping plates 311 on the proximal end 316 side.

Further, a mechanism for positioning when the imaging device 200 is inserted from the proximal end 316 side is disposed on the proximal end 316 side of the main body 312. Specifically, the main body 312 includes a pair of grooves 319 formed in a predetermined length in a direction perpendicular to the outer periphery of the main body 312, pieces 317 thus formed, the engagement protrusions 318 respectively disposed on the pieces 317 and formed to engage with each of the engagement protrusions 217 disposed at the distal end of the grip 202 of the imaging device 200, and the insertion hole 330 into which the positioning protrusion 218 of the grip 202 is inserted.

The engagement protrusions 318 are disposed at a position corresponding to each of the engagement protrusions 217 disposed on the grip 202 of the imaging device 200 to protrude in a direction toward an interior of the main body 312. Accordingly, in the present embodiment, four engagement protrusions 318-1 to 318-4 are arranged at positions corresponding to the four engagement protrusions 217-1 to 217-4. Further, the insertion hole 330 is disposed at a position corresponding to the positioning protrusion 218 disposed on the grip 202 of the imaging device 200. Accordingly, in the present embodiment, one insertion hole 330 is disposed at a position corresponding to one positioning protrusion 218.

### 5. Use states of imaging device 200 and assistance tool 300

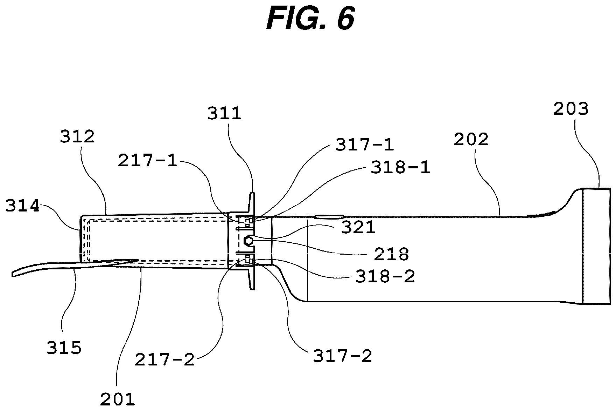

Fig. 6 is the schematic view illustrating the use states of the imaging device 200 and the assistance tool 300 according to one embodiment of the present disclosure. Hereinafter, a case where the imaging device 200 is inserted into the assistance tool 300 will be described with reference to Fig. 6. First, if the imaging device 200 is inserted into the proximal end 316 through the opening 320 of the assistance tool 300 and close to the distal end 314, the engagement protrusions 217 formed to protrude outward from the grip 202 of the imaging device 200 and the engagement protrusions 318 formed toward the interior of the main body 312 of the assistance tool 300 are in contact with each other. Here, in the present embodiment, the engagement protrusions 318-1 to 318-4 of the main body 312 are formed on the pieces 317-1 to 317-4 formed by a pair of grooves 319-1a and 319-1b to 319-4a and 319-4b.
Accordingly, if a force is further applied in a direction where the imaging device 200 is inserted in a state where the engagement protrusions 217 of the imaging device 200 and the engagement protrusions 318 of the assistance tool 300 are in contact with each other, the pieces 317 formed by the pair of grooves 319 are lifted up. In this way, the engagement protrusions 217 of the imaging device 200 are further inserted beyond the engagement protrusions 318 of the assistance tool 300.

Next, if the imaging device 200 is inserted beyond the engagement protrusions 318 of the assistance tool 300, the positioning protrusion 218 disposed on the grip 202 of the imaging device 200 to protrude outward is inserted into the insertion hole 330 of the assistance tool 300. Then, the positioning protrusion 218 of the imaging device 200 is in contact with a wall surface of the insertion hole 330 formed on the distal end 314 side. In this way, the imaging device 200 is restricted without being further inserted in a direction of the distal end 314 of the assistance tool 300.

In other words, in the present embodiment, in a state where the imaging device 200 is completely inserted into the assistance tool 300, movement of the imaging device 200 in the direction where the imaging device 200 is inserted is restricted by the positioning protrusion 218 and the insertion hole 330, and movement of the imaging device 200 in a direction opposite to the direction, that is, in a direction where the imaging device 200 is removed is restricted by the engagement protrusions 217 and the engagement protrusions 318. Such positioning protrusion 218 and insertion hole 330 are installed, in a circumferential direction of the imaging device 200 and the assistance tool 300, preferably at one location or asymmetrically in a case of being installed at a plurality of locations. With such installation, it is possible to restrict a circumferential position and movement of the assistance tool 300 with respect to the imaging device 200. In other words, an up-and-down direction of the imaging device 200 and an up-and-down direction of the assistance tool 300 can be always kept constant, and for example, the tongue depressor 315 can be installed to be always located below the display 203 of the imaging device 200.

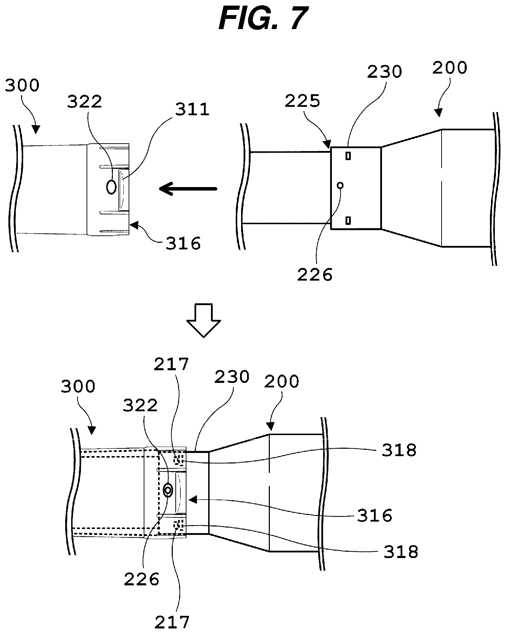

Here, Fig. 7 is the schematic view illustrating how the imaging device 200 and the assistance tool 300 are attached according to one embodiment of the present disclosure. Specifically, Fig. 7 is a view illustrating a case where the imaging device 200 is inserted into the assistance tool 300 by enlarging a surrounding area of the proximal end 316 of the assistance tool 300 and a surrounding area of the connecting portion 230 of the imaging device 200. According to Fig. 7, a notifier 226 (not illustrated in Fig. 6 and the like) is included on an upper surface of the connecting portion 230 of the imaging device 200. Examples of such notifier 226 include a light emitter such as a position or a plurality of LEDs. The notifier 226 emits light if power of the imaging device 200 is turned on by pressing down the power button 221 of the imaging device 200, and stops emitting the light if the power is turned off by pressing down the power button 221 again. Note that, in an example of Fig. 7, the notifier 226 is disposed on the upper surface of the connecting portion 230, and a position of the notifier 226 may be disposed at any position as long as the position is covered by the assistance tool 300.

Further, in the assistance tool 300 into which the imaging device 200 having the notifier 226 is inserted, a notification window 322 is included near the gripping plates 311 on the proximal end 316 side. As an example, the notification window 322 may be formed as a through hole penetrating the assistance tool 300 in a thickness direction, or may be made of a material having a higher transmittance of the light emitted from the notifier 226 with respect to a peripheral area thereof. Conversely, in the assistance tool 300, an area excluding the notification window 322 and the cover 313 (Fig. 4), that is, at least an area around the notification window 322 may be made of a material that does not transmit light, or may be made of a material having a transmittance lower than that of the notification window 322 to inhibit transmission of at least a part of the light from the notifier 226.

Moreover, in a state where the imaging device 200 is inserted from the opening of the assistance tool 300, and the notifier 226 and the notification window 322 are positioned with respect to each other by the engagement protrusions 217 of the imaging device 200 and the engagement protrusions 318 of the assistance tool 300, the notifier and the notification window are positioned to vertically overlap with each other. In other words, in such state, the notification window 322 of the assistance tool 300 is disposed at a position facing a disposition position of the notifier 226 of the imaging device 200, whereby the light emitted from the notifier 226 can be visually recognized through the notification window 322. In this way, the user can visually check that the assistance tool is attached to the imaging device 200.

Note that, in the example of Fig. 7, a case where the notifier 226 is formed by the LED has been described. However, the notifier 226 only needs to be able to notify that the imaging device 200 has been inserted into the assistance tool 300 through the notification window 322. In other words, the notifier 226 can be formed by a simply colored portion or can be formed by a character, a pattern, or the like.

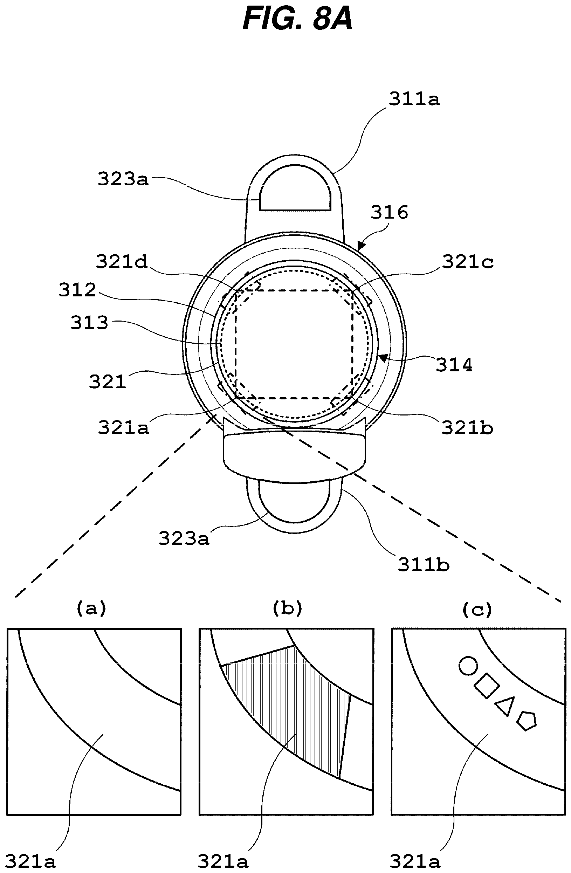

Fig. 8A is the schematic view illustrating the configuration of the assistance tool 300 in the front view according to one embodiment of the present disclosure. Specifically, Fig. 8A is a view for illustrating a structure of the assistance tool 300 and a relationship between an angle of view of the imaging device 200 and a cover collar of the assistance tool 300 in a case where the assistance tool 300 is viewed from the front.

According to Fig. 8A, the assistance tool 300 includes the pair of gripping plates 311 (a gripping plate 311a and a gripping plate 311b) extended outward in the direction where the assistance tool 300 is inserted into the oral cavity from the main body 312 at the proximal end 316 which is an end portion in a direction opposite to the direction. The gripping plates 311 may include through holes 323 (a through hole 323a and a through hole 323b) penetrating in the thickness direction thereof, that is, in the direction where the assistance tool 300 is inserted into the oral cavity. As an example, such through holes 323 are formed in a shape along outer shapes of the gripping plates 311, but naturally, the through holes are not limited to such shape. For example, the through holes may be formed by one or a plurality of small holes, or may be formed in other shapes. By disposing such through holes 323 in the gripping plates 311, for example, even if the entire assistance tool 300 is inserted into the oral cavity, the subject person does not inhibit respiration due to presence of the through holes 323.

Further, the assistance tool 300 includes the cover 313 through which the light detected by the imaging device 200 can pass at the distal end 314 which is the end portion in the direction where the assistance tool 300 is inserted into the oral cavity. Such cover 313 is formed along the cross-sectional shape of the main body 312 in a direction perpendicular to a direction where the assistance tool 300 is inserted into the oral cavity. Note that naturally, the shape is not limited to a shape along the cross-sectional shape of the main body 312. Any shape may be used as long as the shape does not excessively inhibit the imaging of the subject image by the imaging device 200.

The assistance tool 300 includes a cover collar 321 that is disposed around the cover 313 and connects the main body 312 and the cover 313, so that such cover 313 is disposed at the end portion in the direction where the assistance tool 300 is inserted into the oral cavity. A shape of the cover collar on a side in contact with the main body 312 is formed in a shape along the cross section of the main body 312, and the shape of the cover collar on a side in contact with the cover 313 is formed in a shape along an outer shape of the cover 313. Such cover collar 321 is not necessarily formed as a component clearly distinguishable from the main body 312 or the cover 313, and may refer to an area in contact with the cover 313 as a portion of the main body 312 or an area in contact with the main body 312 as a part of the cover 313.

In a case where the imaging device 200 is inserted into and positioned with respect to the assistance tool 300, the cover collar 321 has, in at least a part thereof, an appearing-in-image area that is included within an angle of view captured by the imaging device 200. In an example of Fig. 8A, as for the appearing-in-image area, four appearing-in-image areas of an appearing-in-image area 321a to an appearing-in-image area 321d are formed to correspond to four corners of a square angle of view. Note that the appearing-in-image area 321a to the appearing-in-image area 321d are formed at four locations in the example of Fig. 8A, but the present disclosure is not limited thereto, and the appearing-in-image areas may be formed at only one or a plurality of locations.

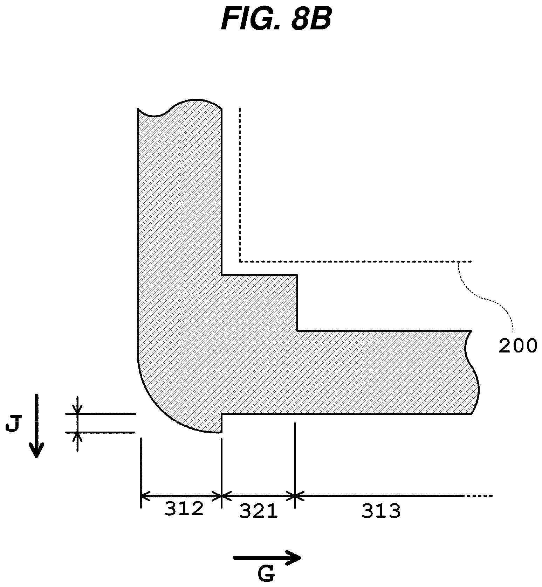

Here, Fig. 8B is the schematic view illustrating the formation of a part of the distal end 314 of the assistance tool 300 in the cross-sectional view according to one embodiment of the present disclosure. Specifically, Fig. 8B is a view schematically illustrating a cross section in the longitudinal direction near the appearing-in-image area 321a illustrated in Fig. 8A. According to Fig. 8B, the assistance tool 300 includes the main body 312 forming an outer shape thereof, the cover 313 formed inside the main body 312 along the shape of the main body 312, and the cover collar 321 connecting the main body 312 and the cover 313. Here, for example, the cover collar 321 is formed to protrude in a direction G from the interior of the main body 312 inward. Therefore, as indicated by a broken line in Fig. 8B, if the imaging device 200 is inserted along an inner wall of the main body 312, the cover collar 321 has a positional relationship in which at least a part of the cover collar faces a surface in a lateral direction (that is, a direction parallel to the direction G) of the imaging device 200. Therefore, if the subject image is captured by the imaging device 200, a facing area of the cover collar 321 is included within the angle of view as the appearing-in-image area 321a.

Note that, as illustrated in Fig. 8B, the cover collar 321 may be suitably formed to protrude along a direction perpendicular to the direction G with respect to an inner surface of the cover 313. With such configuration, it is possible to prevent the distal end of the imaging device 200 from being directly in contact with the cover 313.

Returning to Fig. 8A again, as illustrated in (a), the appearing-in-image area 321a to the appearing-in-image area 321d may be formed to have the same structure as that of the cover collar 321 other than the areas. Further, as illustrated in (b) and (c), the appearing-in-image area 321a to the appearing-in-image area 321d may include a structure formed by a predetermined pattern on at least a part of an inner surface or an outer surface of the cover collar 321 in the direction where the assistance tool 300 is inserted into the oral cavity. In an example of (b), the structure is formed by a plurality of concave grooves or a plurality of convex patterns formed at predetermined intervals. Further, in an example of (c), the structure is formed by a predetermined character string formed in a groove shape. Note that such structures may be formed by any method, such as a concave or convex pattern or a pattern printed on a surface of the structure.

Note that, in examples of Figs. 8A and 8B, a case where the appearing-in-image area 321a to the appearing-in-image area 321d are formed in the facing areas of the cover collar 321 has been described. However, these examples are merely examples, and the present disclosure is not limited to these examples. In the present embodiment, the appearing-in-image areas may be areas that can reflect light used for the imaging such as the light emitted from the imaging device 200, and generate reflected light that can be detected a the sensor of the camera 211. Accordingly, it is sufficient that the assistance tool 300 has any one of the areas, and it is not necessary that the appearing-in-image area 321a to the appearing-in-image area 321d are physically separated from other areas or intentionally formed in advance during processing or molding.

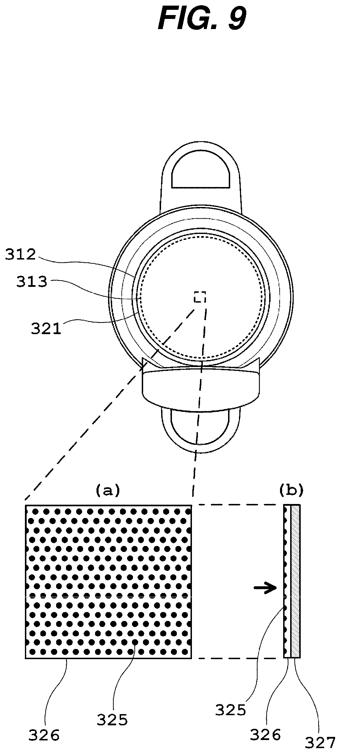

Fig. 9 is the schematic view illustrating the configuration of the assistance tool 300 in the front view according to one embodiment of the present disclosure. Specifically, Fig. 9 is a view for illustrating a surface structure of the cover 313 in a case where the assistance tool 300 is viewed from the front.

According to Fig. 9, the cover 313 formed to be in contact with the interior of the main body 312 via the cover collar 321 is included at the distal end 314 which is the end portion in the direction where the assistance tool 300 is inserted into the oral cavity. The cover 313 is made of a material capable of being passed through by the light emitted from the imaging device 200 into the oral cavity and light reflected by the subject in the direction of the imaging device 200. Here, since the assistance tool 300 is inserted into the oral cavity to cover the imaging device 200, the assistance tool is always affected by exhalation of the subject person. In other words, the surfaces of the cover 313 are fogged by respiration of the subject person, and the fogging may adversely affect the imaging of the subject by the imaging device 200. Therefore, if necessary, an antifogging agent may be laminated on the outer surface or the inner surface of the cover 313 in the direction where the assistance tool 300 is inserted into the oral cavity.

The antifogging agent has a function of imparting an antifogging property by forming a coating layer on the outer surface or the inner surface of the cover 313 by laminating the antifogging agent to improve wettability. Such antifogging agent is not particularly limited to those exemplified below, but an antifogging agent of a siliconbased material, a high-molecular type or low-molecular type hydrophilic material (for example, a nonionic antifogging agent containing, as a main component, sucrose fatty acid ester, glycerin fatty acid ester, polyglycerin fatty acid ester, sorbitan fatty acid ester, polyoxyethylene alkyl ether, polyoxyethylene alkylphenyl ether, a polyoxyethylene polyoxypropylene block copolymer, polyethylene glycol fatty acid ester, polyoxyethylene sorbitan fatty acid ester, polyoxyethylene glycerin fatty acid ester, polyoxyethylene polyglycerin fatty acid ester, fatty acid alkanolamide, or a combination thereof; and an amphoteric antifogging agent containing, as a main component, an alkyl betaine such as lauryldimethylaminoacetic acid betaine or stearyldimethylaminoacetic acid betaine, fatty acid amidopropyl betaine such as cocamidopropyl betaine, alkyl imidazole such as 2-alkyl-N-carboxymethyl-N-hydroxyethylimidazolinium betaine, amino acid such as sodium lauroyl glutamate, amine oxide such as lauryl dimethylamine N-oxide or oleylemethylamine N-oxide, or a combination thereof) typified by a surfactant, an MCP polymer-based antifogging agent, or a combination thereof is appropriately used.

Such antifogging agent may be applied to the cover 313 in advance or may be applied later. Further, the present disclosure is not limited to a case of applying a liquid antifogging agent, and a sheet-like material on which the antifogging agent is laminated in advance may be laminated.

Here, even in a case where the antifogging agent is laminated on the cover 313 or in a case where only the cover 313 is used without laminating the antifogging agent, an iridescent interference pattern may be generated under influence of the light emitted from the imaging device 200, light reflected on the subject, or the like, and may adversely affect the imaging by the imaging device 200. Therefore, as illustrated in (a) and (b) of Fig. 9, the assistance tool 300 including the cover 313 includes a translucent resin layer 327 made of the thermoplastic resin or the like as a base material. Such translucent resin layer 327 is preferably made of a cyclic olefin resin such as an amorphous cyclic olefin resin or an amorphous cyclic olefin resin among thermoplastic resins.

Further, by inserting the cover 313 into the oral cavity, as described above, the assistance tool is affected by the exhalation of the subject person, a smooth water film is formed on the antifogging agent or the surfaces of the cover 313, and thus an iridescent pattern or the like may be generated and adversely affect the imaging. Therefore, it is desirable that irregularities exist at predetermined intervals or randomly on a boundary surface of the antifogging agent or the cover, and the light is diffusely reflected. Specifically, it is desirable to form the irregularities at the predetermined intervals or randomly as described above in a boundary surface between the antifogging agent and the water film formed by the exhalation, a boundary surface between the antifogging agent and the cover 313, a boundary surface between the cover 313 and the water film formed by the exhalation (in a case where the antifogging agent is not applied), or a combination thereof. As an example, a coating layer 326 laminated by applying the antifogging agent to a surface of the base material of the translucent resin layer 327 may be formed on the cover 313, and recesses 325 may be arranged at predetermined intervals or randomly on a surface of the coating layer 326. An interval between two of the recesses 325 is, on average, an interval wider than a wavelength band of the light detected by the imaging device 200 (for example, a wavelength band of red light of 830 nm or more), and more preferably an interval wider than twice the wavelength band. By forming the recesses 325 at such intervals, it is possible to prevent the generation of the interference pattern due to diffuse reflection of the light.

Further, a thickness of the coating layer 326 can be adjusted based on a coherence length calculated from the wavelength band of the light detected by the imaging device 200, and is preferably 10 um or more as an example.

Note that, in an example of Fig. 9, a case where the coating layer 326 is formed on an outer surface of the translucent resin layer 327 has been described, but such coating layer 326 may be formed on an inner surface of the translucent resin layer 327. Further, in the example of Fig. 9, a case where the recesses 325 are formed in the coating layer 326 has been described, but the recesses 325 may be directly formed in the translucent resin layer 327 by a method of subjecting a surface of the translucent resin layer 327 to an acid treatment or a plasma treatment or printing a pattern on the surface. Further, in the example of Fig. 9, a case where the cover 313 is formed by the coating layer 326 and the translucent resin layer 327 has been described, but layers other than these layers may be further included.

Further, in Fig. 8B, a part of a distal end portion of the main body 312 protrudes from the outer surface of the cover 313 in a direction J perpendicular to the direction G from the inner surface of the main body 312 to the inside. With such configuration, in a case where the antifogging agent is applied or laminated in Fig. 9, it is possible to prevent the antifogging agent from leaking beyond the protruding distal end portion.

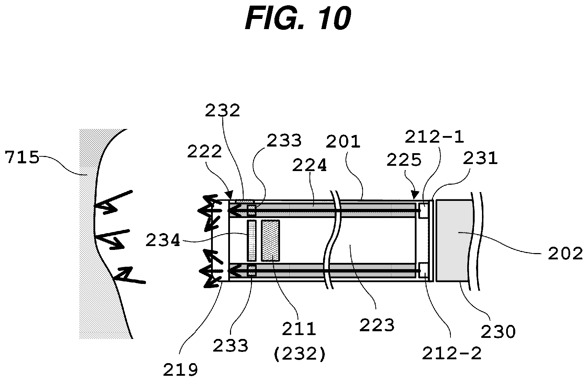

Here, Fig. 10 is the schematic view illustrating the configuration of the imaging device 200 in the cross-sectional view according to one embodiment of the present disclosure. Specifically, Fig. 10 is a view schematically illustrating a process of generating the subject image in the imaging device 200 and illustrating another example for suppressing the generation of the interference pattern. According to Fig. 10, as for the light sources 212, four light sources 212-1 to 212-4 in total are disposed on a substrate 231 disposed on a distal end side of the grip 202. As an example, each of the light sources 212 includes the LED, and light having a predetermined frequency band is emitted from the LED toward the oral cavity. The light emitted from the light sources 212 enters the proximal end 225 of the main body 201, and is guided toward the diffusion plate 219 by the wall portion 224 of the main body 201. The light reaching the diffusion plate 219 is diffused into the oral cavity by the diffusion plate 219. Then, the light diffused by the diffusion plate 219 is reflected by the pharynx 715 or the like that is the subject. When the reflected light reaches the camera 211, the subject image is generated.

At this time, the wall portion 224 of the main body 201 has a function of guiding the light emitted from the light sources 212-1 to 212-4 to the diffusion plate 219 as described above, and may have a first polarizing plate 233 at a part or an end portion of the wall portion. Further, the sensor of the camera 211 is disposed in the accommodation space 223 formed by the wall portion 224 of the main body 201, and the imaging device 200 may have a second polarizing plate 234 on the subject side than the sensor. At this time, the first polarizing plate 233 and the second polarizing plate 234 are arranged to make their polarization axes orthogonal to each other at 90°. In this way, it is possible to suppress reflection of the light sources 212-1 to 212-4 due to regular reflected light (linearly polarized light) generated when light incident on the subject via the first polarizing plate 233 is reflected by the subject, and it is possible to obtain a better subject image.

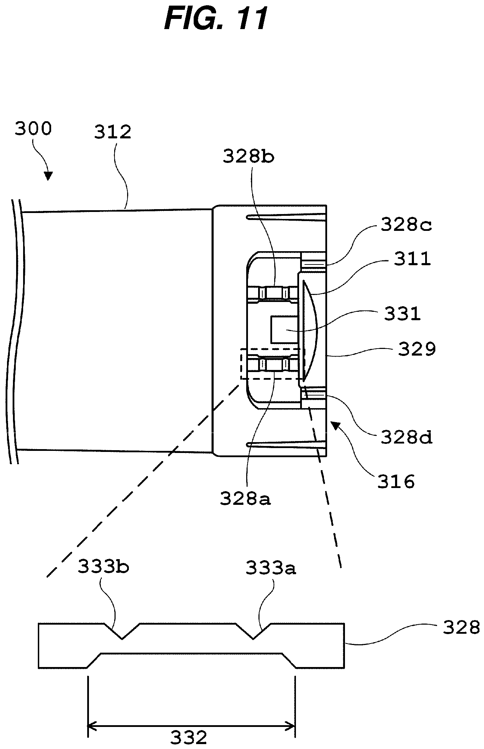

Fig. 11 is the schematic view illustrating the configuration of the assistance tool 300 in the top view according to one embodiment of the present disclosure. Specifically, Fig. 11 is a view schematically illustrating an example of another structure of the assistance tool 300 by enlarging the surrounding area of the proximal end 316 of the assistance tool 300. According to Fig. 11, the assistance tool 300 may include a positioning mechanism 329 that is deformable or separable from the main body 312 by applying a load in a predetermined direction.

The positioning mechanism 329 includes the gripping plate 311 formed to extend toward the outer side of the main body 312 in a direction perpendicular or substantially perpendicular to the direction where the assistance tool 300 is inserted into the oral cavity, a positioning piece 331 for positioning the imaging device 200 and the assistance tool 300, and connecting pieces 328 (a connecting piece 328a to a connecting piece 328d) for connection to the main body 312. Each of the connecting pieces 328 has one end connected to a main body portion of the positioning mechanism 329, and the other end connected to the main body 312, thereby connecting the positioning mechanism 329 and the main body 312. Here, as illustrated in Fig. 11, each of the connecting pieces 328 includes, in a cross section in a longitudinal direction, a notch 333a and a notch 333b formed in a groove shape along a lateral direction, and a thin-walled area 332 at least partially formed thin in a thickness direction. Therefore, the connecting pieces 328 are formed to be easily deformed or easily cut in a case where a load is applied in a predetermined direction.

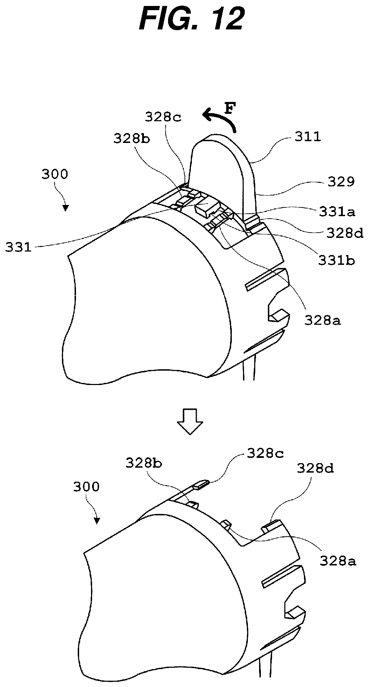

Here, Fig. 12 is the schematic view illustrating the configuration of the positioning mechanism 329 of the assistance tool 300 according to one embodiment of the present disclosure. Specifically, Fig. 12 is a view for illustrating a structure and a use method of the positioning mechanism 329 illustrated in Fig. 11 in more detail. According to Fig. 12, the positioning piece 331 of the positioning mechanism 329 includes an inclined surface 331a inclined from its end portion toward its interior and a recess 331b. In other words, if the imaging device 200 is inserted into the assistance tool 300, a projection (not illustrated) formed on the upper surface of the connecting portion 230 of the imaging device 200 is inserted while being in contact with the inclined surface 331a, whereby the positioning mechanism 329 rises upward. The imaging device 200 is further inserted without change and the projection of the imaging device 200 is finally fitted into the recess 331b of the positioning piece 331, whereby the assistance tool 300 and the imaging device 200 are positioned with respect to each other.

If the imaging device 200 is inserted into the assistance tool 300 and positioned by the positioning piece 331, it is difficult to remove the assistance tool 300 from the imaging device 200 due to fitting between the projection on the imaging device 200 side and the recess 331b of the positioning piece 331. Therefore, the user applies a load to the positioning mechanism 329 by pushing the gripping plates 311 of the positioning mechanism 329 in a direction F. Accordingly, the connecting piece 328a to the connecting piece 328d in which the notch 333a and the notch 333b and the thin-walled area 332 are formed are deformed or cut, whereby the positioning mechanism 329 connected to the main body 312 rises upward, and the fitting between the projection on the imaging device 200 side and the recess 331b of the positioning piece 331 is released.

Fig. 12 illustrates a state where each of the connecting pieces 328 is cut by applying the load to the positioning mechanism 329 and the positioning mechanism 329 is separated from the main body 312. In such state, even if the imaging device 200 is inserted into the assistance tool 300 again, the imaging device 200 cannot be positioned with respect to the assistance tool 300, since the positioning mechanism 329 including the positioning piece 331 has already been separated. Therefore, the user cannot attach the assistance tool 300 on the imaging device 200, and the imaging device 200 can be prevented from being inserted again into the assistance tool 300 once inserted.

Note that, in an example of Fig. 12, a case where the connecting pieces 328 are deformed or cut by applying a load to the positioning mechanism 329 from a predetermined direction has been described, but the connecting pieces may be deformed or cut using a tool such as scissors or a nipper, for example.

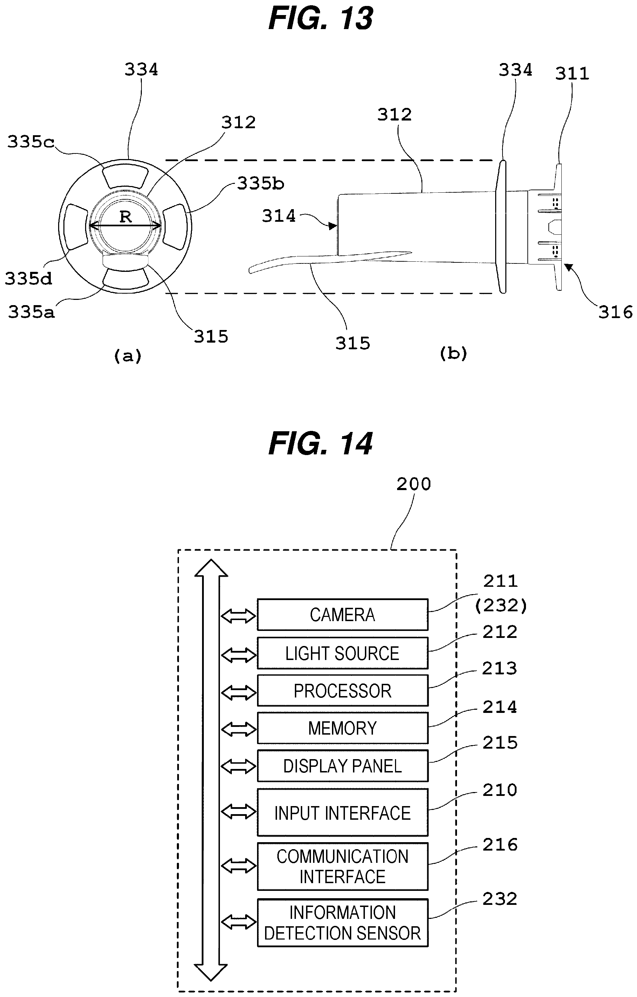

Fig. 13 is the schematic view illustrating the formation of the plate 334 of the assistance tool 300 according to one embodiment of the present disclosure. Specifically, (a) of Fig. 13 is a front view of the assistance tool 300, and (b) is a side view of the assistance tool 300. According to Fig. 13, the assistance tool 300 can include the plate 334 so as to be along the outer periphery of the main body 312. By installing the plate 334 on the outer periphery of the main body 312, in a case where the assistance tool 300 and the imaging device 200 are inserted more than necessary, the plate 334 is coming into contact with teeth or the lips, and make it possible to prevent the assistance tool 300 and the imaging device 200 from being excessively inserted into the back of the throat.

According to Fig. 13, the plate 334 is formed in an annular shape along the outer periphery of the main body 312. The plate 300 is installed on the proximal end 316 side of the assistance tool 300 at a position corresponding to a distance from the distal end of the tongue depressor 315 to the lips or the incisors of the subject person. Further, the plate 334 is disposed to protrude from the main body 312 toward its outer peripheral direction in a direction perpendicular or substantially perpendicular to the direction where the assistance tool 300 is inserted into the oral cavity. At this time, it is desirable that the plate 334 has a height corresponding to a size of a mouth of the subject person in a case where the subject person opens the mouth from the main body 312 in the outer peripheral direction. By forming the plate according to these distances and heights, it is possible to effectively prevent excessive insertion of the assistance tool 300 and the imaging device 200. Note that a shape of the plate 334 is merely an example, and the plate is not necessarily formed in an annular shape as long as at least a part of the plate extends along the outer periphery of the main body 312. Further, in such case, the plate 334 does not need to be only one along the outer periphery of the main body 312, but more than one plate 334 may be installed.

Further, according to Fig. 13, the plate 334 may include one or a plurality of through holes 335a to 335d. As an example, the through holes are formed to penetrate in the thickness direction in a manner of following an outer shape of the plate 334, but are naturally not limited to such shape. For example, the through holes may be formed by one or a plurality of small holes, or may be formed in other shapes. Further, in an example of Fig. 13, four through holes 335a to 335d are exemplified as the through holes, but naturally, the number and arrangement positions are not limited thereto. For example, the small holes may be disposed entirely, or one large hole may be installed in one location. By disposing such through holes 335a to 335d in the plate 334, for example, even if the entire assistance tool 300 is inserted into the oral cavity, the assistance tool 300 does not inhibit respiration of the subject person due to presence of the through holes 335a to 335d.

Note that, in the example of Fig. 13, a case where the plate 334 is integrally molded in advance during manufacturing of the assistance tool 300 has been described. However, the present disclosure is not limited thereto, and the plate 334 may be formed in an annular shape (a doughnut shape) to be detachable from the assistance tool 300. In the present embodiment, the assistance tool 300 is formed to have a diameter gradually decreasing from the proximal end 316 toward the distal end 314. Therefore, by preparing a plurality of plates 334 having different inner diameters R, the plates 334 can be installed at positions corresponding to a distance of a depth in the oral cavity of the subject person, an age of the subject person, the size of the mouth of the subject person, and the like.

### 6. Processes in Imaging Device 200

Fig. 14 is the block diagram illustrating the configuration of the imaging device 200 according to one embodiment of the present disclosure. According to Fig. 14, the imaging device 200 includes the camera 211, the light source 212, a processor 213, a memory 214, a display panel 215, an input interface 210, and a communication interface 216. These components are electrically connected to each other via a control line and a data line. Note that the imaging device 200 does not need to include all of the components illustrated in Fig. 14, and can also be configured without some of the components, or can also be configured by adding other components. For example, the imaging device 200 can include a battery for driving each of the components, and the like.

In the imaging device 200, the camera 211 functions as an imaging unit that generates the subject image by detecting the reflected light reflected on the oral cavity which is the subject. In order to detect the light, the camera 211 includes, as an example, a CMOS image sensor, a lens system, and a drive system for implementing a desired function. The image sensor is not limited to the CMOS image sensor, and other sensors such as a CCD image sensor can also be used. Further, it is also possible to additionally use an imaging element according to a band of the reflected light to be detected. Although not specifically illustrated, for example, the camera 211 can have an autofocus function, and it is preferable that a focus of the camera be set, for example, on the front of the lens to match a specific site. In the camera, the CMOS image sensor, which is the imaging element that detects a wavelength in a near-infrared light band from a wavelength in a visible light band, and a sensor, which is an infrared light imaging element that detects a wavelength in an infrared light band may be arranged adjacent to each other on an image sensor substrate. Further, the camera 211 can have a zoom function, and is preferably set to performing the imaging at an appropriate magnification according to a size of the pharynx or the influenza follicle.

Here, it has been known that there is the specific pattern for the influenza in the lymphatic follicles appearing at the deepest part of the pharynx located inside the oral cavity. The lymphatic follicles having this specific pattern are called the influenza follicles, which are the characteristic sign of the influenza, and are said to appear about 2 hours after the onset. As described above, the processing system 1 of the present embodiment is used to determine the possibility of the subject person contracting the influenza by imaging the pharynx of the oral cavity and detecting the above follicles, for example. Therefore, if the imaging device 200 is inserted into the oral cavity, a distance between the camera 211 and the subject becomes relatively short. Accordingly, the camera 211 preferably has an angle of view (2θ) at which a value calculated by [(distance from the distal end portion of the camera 211 to a rear wall of the pharynx) * tanθ] is 20 mm or more in a vertical direction and 40 mm or more in a horizontal direction. By using the camera having such angle of view, even if the camera 211 and the subject are close to each other, it is possible to image a wider range. In other words, as the camera 211, a normal camera can be used, but a camera called a wide-angle camera or a super-wide-angle camera can also be used.

The light source 212 is driven by an instruction from the processing device 100 or the imaging device 200, and functions as a light source unit for irradiating the oral cavity with the light. The light source 212 includes one or a plurality of light sources 212. In the present embodiment, the light source 212 includes one or a plurality of LEDs, and light having a predetermined frequency band is emitted from each of the LEDs in a direction of the oral cavity. As the light source 212, light having a desired band among an ultraviolet light band, a visible light band, and an infrared light band, or a combination thereof is used. In a case of irradiating a plurality of bands, the plurality of LEDs set in advance to emit the light in each of the bands are switched to display.

The processor 213 functions as a control unit that controls other components of the imaging device 200 based on a program stored in the memory 214. The processor 213 controls driving of the camera 211 and driving of the light source 212 based on the program stored in the memory 214, and controls storage of the subject image captured by the camera 211 in the memory 214. Specifically, the processor 213 executes, a process of starting capturing the subject image by receiving an operation input by the user, a process of determining whether or not at least a part of the cover collar 321 of the assistance tool 300 is included in the subject image based on the captured subject image, a process of outputting information indicating that the imaging device 200 is not inserted into the assistance tool 300 in a case where a determination result is that at least a part of the cover collar 321 of the assistance tool 300 not included, and the like. The processor 213 mainly includes one or a plurality of CPUs, and may be appropriately combined with other processors.

The memory 214 includes a RAM, a ROM, a nonvolatile memory, an HDD, and the like, and functions as a storage unit. The memory 214 stores, as a program, instruction commands for various control operations of the imaging device 200. Specifically, the memory 214 stores a program for executing the process of starting capturing the subject image by receiving the operation input by the user, the process of determining whether or not at least a part of the cover collar 321 of the assistance tool 300 is included in the subject image based on the captured subject image, the process of outputting information indicating that the imaging device 200 is not inserted into the assistance tool 300 in the case where a determination result is that at least a part of the cover collar 321 of the assistance tool 300 is not included, and the like.

The display panel 215 is disposed on the display 203, and functions as a display unit for displaying the subject image captured by the imaging device 200 and the information indicating that the imaging device 200 is not inserted into the assistance tool 300. The display panel 215 includes a liquid crystal panel, but is not limited to the liquid crystal panel, and may include an organic EL display, a plasma display, or the like.

The input interface 210 functions as an input unit that receives an instruction input of the subject person to the processing device 100 and the imaging device 200. Examples of the input interface 210 include physical key-buttons such as a "capture button" for instructing start/end of recording by the imaging device 200, a "power button" for turning on/off the power of the imaging device 200, a "confirmation button" for performing various selection operations, a "return/cancel button" for returning to a previous screen or canceling an input confirmation operation, and a cross key-button for moving an icon or the like displayed on the display panel 215. Note that these various buttons/keys may be physically prepared, or may be displayed as an icon on the display panel 215 and selectable using a touch panel or the like arranged as the input interface 210 in a superimposed manner on the display panel 215. A method of detecting the instruction input of the subject person by the touch panel may be any method such as a capacitance type or a resistive film type.

The communication interface 216 functions as a communication unit for transmitting and receiving information to and from the processing device 100 and/or other devices. Examples of the communication interface 216 include various elements, for example, a connector for wired communication such as a USB and an SCSI, a transmission/reception device for wireless communication such as a wireless LAN, Bluetooth (registered trademark), and an infrared ray, and various connection terminals for a printed mounting board and a flexible mounting board.

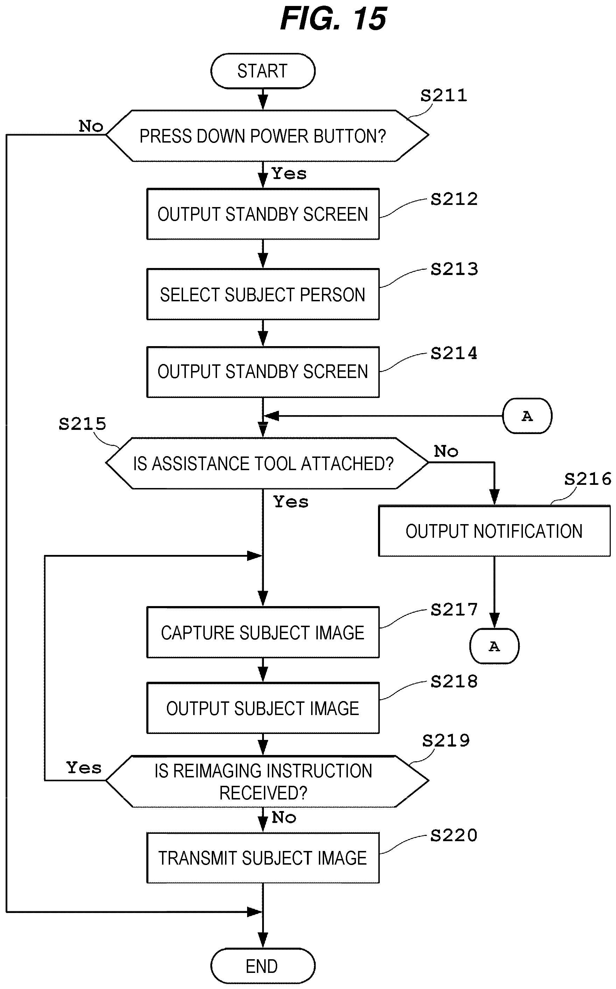

Fig. 15 is the chart illustrating the processing flow executed in the imaging device 200 according to one embodiment of the present disclosure. The processing flow is mainly performed by the processor 213 of the imaging device 200 reading and executing the program stored in the memory 214.

According to Fig. 15, the processor 213 determines whether or not the operation input by the user is received via the input interface 210 (for example, the power button) (S211). Note that at this time, if the processor 213 determines that the input by the user is not received, the processing flow ends.

Meanwhile, if the processor 213 determines that the input by the user is received, the processor outputs a standby screen to the display panel 215 (S212). The standby screen (not illustrated) includes a through image captured via the camera 211. Then, the processor 213 receives the operation input by the user via the input interface 210, and receives selection of information for specifying the subject person with the oral cavity to be imaged (S213). If the subject person to be imaged is selected, the processor 213 outputs the standby screen to the display panel 215 again (S214).

Next, the user covers the distal end of the imaging device 200 with the assistance tool 300, and inserts the imaging device 200 into the oral cavity of the subject person to a predetermined position. Then, the processor 213 determines whether or not the assistance tool 300 is attached to the imaging device 200 based on the subject image (the through image) captured by the camera 211 (S215). For example, the determination is performed by inputting the captured subject image to a learned assistance tool attachment determination model stored in the memory 214. Note that use of the learned assistance tool attachment determination model for the determination is merely an example, and the processor may determine whether or not at least the part of the cover collar is included in the subject image by a rule-based image analysis process, and may determine whether or not the assistance tool 300 is attached to the imaging device 200. Further, it is not necessary for the processor 213 of the imaging device 200 to execute the process in both a case of using the learned assistance tool attachment determination model and a case of using the rule-based image analysis process. In other words, the process related to the determination may be made by transmitting the subject image captured by the imaging device 200 to a server device connected via a network, and applying the subject image to the learned assistance tool attachment determination model or the rule-based image analysis process in the server device, and the imaging device 200 may receive a determination result from the server device.

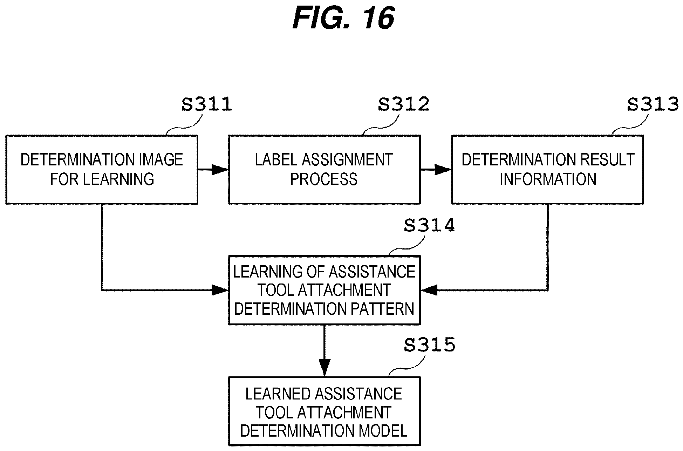

Here, Fig. 16 is the view illustrating the example of the screen output in the imaging device 200 according to one embodiment of the present disclosure. Specifically, Fig. 16 is a view illustrating a processing flow related to generation of the learned assistance tool attachment determination model used in S215 of Fig. 15. The processing flow may be executed by the processor 213 of the imaging device 200 or may be executed by a processor of another processing device connected by communication.

According to Fig. 16, the processor acquires the subject image as a determination image for learning by the camera 211 of the imaging device 200 (S311). Next, the processor executes a processing step of assigning a correct answer label to the determination image for learning based on a determination result as to whether or not the image includes at least a part of the cover collar 321 of the assistance tool 300 reflected by the user or the like in advance (S312). Then, the processor stores assigned correct answer label information in association with the determination image for learning used for assigning the correct answer label (S313).

Once the determination image for learning and the correct answer label information associated with the determination image for learning are respectively obtained, the processor executes a step of performing machine learning of an attachment determination pattern of the assistance tool 300 using the determination image for learning and the correct answer label information (S314). As an example, the machine learning is performed by giving a set of these pieces of information to a neural network in which neurons are combined, and repeating learning while adjusting parameters of each of the neurons to make an output from the neural network become the same as the correct answer label information. Then, a step of acquiring the learned assistance tool attachment determination model is executed (S315). The acquired assistance tool attachment determination model is stored in the memory 214 of the imaging device 200 or another processing device connected to the imaging device 200 via a wired or wireless network. In this way, by using the learned assistance tool determination model, it is possible to determine whether or not the subject image captured by the camera 211 is the image in which at least a part of the cover collar 321 of the assistance tool 300 is included.

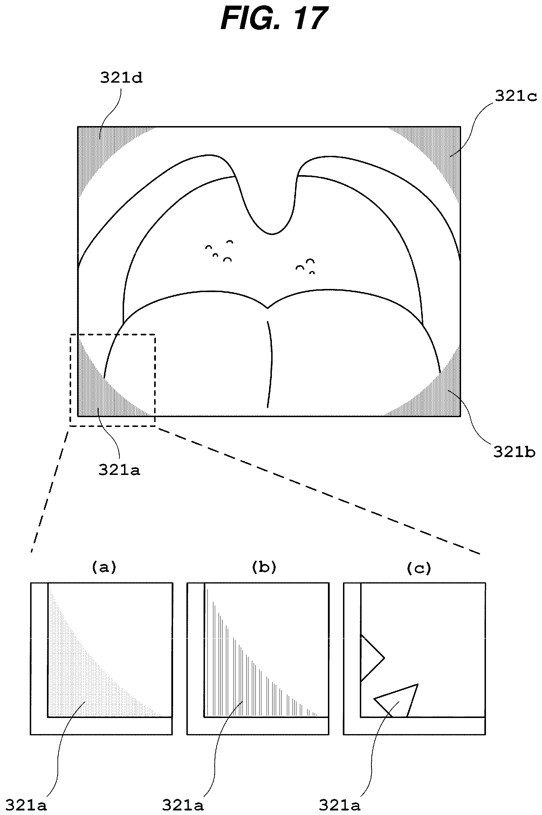

Further, Fig. 17 is the view illustrating the example of the screen output in the imaging device 200 according to one embodiment of the present disclosure. Specifically, Fig. 17 is a view illustrating an example of a subject image used for determination in S215 of Fig. 15. According to Fig. 17, the subject image captured by the camera 211 of the imaging device 200 includes the appearing-in-image area 321a to the appearing-in-image area 321d of the cover collar 321 included, in addition to the image of the oral cavity as the subject, at four corners of the image. In other words, the light emitted from the light source 212 of the imaging device 200 is reflected by the reflection area 321a to the appearing-in-image area 321d of the cover collar 321, and detected by the camera 211 of the imaging device 200. In this way, these areas are included in the subject image. In this way, if the assistance tool 300 is correctly attached to the imaging device 200, as illustrated in Fig. 17, the appearing-in-image area 321a to the appearing-in-image area 321d of the cover collar 321 are included in the four corners. Accordingly, it is possible to determine whether or not the assistance tool 300 is attached to the imaging device 200 by determining whether or not the appearing-in-image area 321a to the appearing-in-image area 321d of the cover collar 321 are included in the subject image captured by the camera 211 of the imaging device 200.

Note that, as illustrated in Fig. 8A, the appearing-in-image area 321a to the appearing-in-image area 321d of the cover collar 321 can include a structure having a predetermined pattern as necessary. For example, in a case where other areas of the cover collar 321 have the same structure as illustrated in (a) of Fig. 8A, a predetermined pattern is not drawn in the captured image as illustrated in (a) of Fig. 17. Meanwhile, in a case where the cover collar 321 includes a structure formed by a predetermined pattern as illustrated in (b) or (c) of Fig. 8A, the structure is also drawn in addition to the cover collar 321 as illustrated in (b) or (c) of Fig. 17. By using such structure, it is possible to more accurately determine the appearance of the cover collar 321 in the subject image.

Further, in an example of Fig. 17, for convenience of description, the appearing-in-image area 321a to the appearing-in-image area 321d included in the subject image are described to be clearly divided and displayed with respect to other areas. However, distances from the camera 211 to the oral cavity, which is the subject, and the appearing-in-image area 321a to the appearing-in-image area 321d of the cover collar 321 are naturally different. Therefore, depending on settings during the imaging, the appearing-in-image area 321a to the appearing-in-image area 321d may be detected and output without being clearly distinguished as illustrated in Fig. 17, and may also be detected and output as a so-called blurred image.

Returning to Fig. 15 again, in a case where it is determined that the assistance tool 300 is not attached as a result of determined whether or not at least a part of the cover collar is included in the subject image by the learned assistance tool attachment determination model and determining whether or not the assistance tool 300 is attached to the imaging device 200, the processor 213 outputs a notification indicating that the imaging device 200 is not inserted into the assistance tool 300 on the display of the display panel 215 (S216).

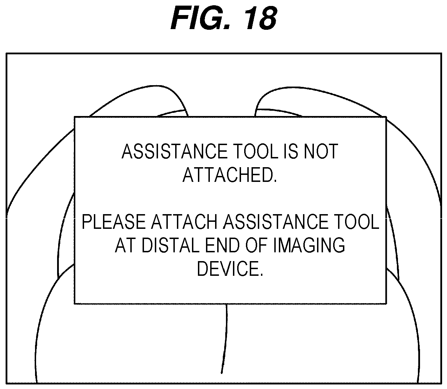

Here, Fig. 18 is the view illustrating the example of the screen output in the imaging device 200 according to one embodiment of the present disclosure. Specifically, Fig. 18 is a view illustrating an example of a notification output to the display of display panel 215 in S216 of Fig. 15. According to Fig. 18, in addition to the subject image captured by the camera 211 of the imaging device 200 as the through image, the notification is output to indicate that the imaging device 200 is not inserted into the assistance tool 300, stating "The assistance tool is not attached. Please attach the assistance tool to the distal end of the imaging device". By outputting such notification, the user can reliably know that the assistance tool 300 is not attached, and the imaging can be performed more smoothly.

Note that, in an example of Fig. 18, a case where such notification is output to the display has been described, but the notification may be provided by another method. For example, the notification may be output to the processing device 100 or another device connected via communication and displayed via these devices. Further, for example, in a case where the imaging device 200 includes an output unit such as a speaker, the notification may be made by voice via the output unit. Further, for example, in a case where the imaging device 200 includes a vibrator, an LED, or the like, the notification may be made by vibration or light emission. Moreover, any notification may be made as long as "the notification indicates that the imaging device 200 is not inserted into the assistance tool 300", and such notification may be a notification indicating that the imaging device is inserted or a notification prompting the insertion.

Returning to Fig. 15 again, in a case where it is determined that the assistance tool 300 is attached to the imaging device 200, the processor 213 receives an imaging start operation by the user via the input interface 210 (for example, the capture button). In this way, the processor 213 controls the camera 211 to start capturing the subject image of the subject (S217). The capture of the subject image is performed by pressing down the capture button to collectively capture a certain number (for example, 30 sheets) of images at regular intervals. If the capture of the subject image ends, the processor 213 stores the captured subject image in association with subject person ID information read in the memory 214. Then, the processor 213 outputs the subject image stored in the memory 214 to the display panel 215 (S218).

Here, the user can take out the imaging device 200 from the oral cavity together with the assistance tool 300, check the subject image output to the display panel 215, and input a reimaging instruction in a case where the desired image is not obtained. Therefore, the processor 213 determines whether or not the input of the reimaging instruction by the user is received via the input interface 210 (S219). In a case where the input of the reimaging instruction is received, the processor 213 displays the standby screen in S215 again to enable the capture of the subject image.

Meanwhile, in a case where the input of the reimaging instruction is not received, the imaging device 200 is returned to the mounting table 400 by the user, and an instruction to finish the imaging is received from the processing device 100, the processor 213 transmits the subject image stored in the memory 214 and the subject person ID information associated with the subject image to the processing device 100 via the communication interface 216 (S220). Thus, the processing flow ends.

Note that, although not particularly illustrated, if the subject image captured in this way is transmitted to the processing device 100, the processor of the processing device 100 selects the determination image to be used for the determination, and determines the possibility of contracting the predetermined disease using the determination image. Then, a determination result is output to the display or the like. Further, this determination may be made by the rule-based image analysis process, or may be made by using a learned determination model obtained by machine learning using a subject image for learning in which disease information such as the influenza is associated as the correct answer label.

In this way, in the present embodiment, it is possible to provide the assistance tool that is suitable for imaging the oral cavity of the subject person and assists in the imaging, and the imaging device, the program, and the method for imaging the inner side of the oral cavity using the assistance tool.

### 7. Variations

In the example of Fig. 15, a case of determining whether or not the assistance tool 300 is attached to the imaging device 200 based on the captured subject image by the processor 213 of the imaging device 200 has been described. However, the present disclosure is not limited thereto, and the determination may be made by another device (for example, the processing device 100, or a server device on a cloud) communicably connected to the imaging device 200, and the imaging device 200 may receive and output a determination result.

Note that these variations are similar to the configurations, the processes, and the procedures in one embodiment described with reference to Figs. 1 to 18, except for points specifically described above. Accordingly, detailed description of these matters will be omitted. Further, it is also possible to configure the system by appropriately combining or replacing each of the elements described in each of the variations or each of the embodiments.

The processes and the procedures described in the present description can also be implemented not only by those explicitly described in the embodiments but also by software, hardware, or a combination thereof. Specifically, the processes and the procedures described in the present description are implemented by installing logic corresponding to the processes on a medium such as an integrated circuit, a volatile memory, a nonvolatile memory, a magnetic disk, or an optical storage. Further, the processes and the procedures described in the present description can be implemented as a computer program and executed by various computers including the processing device and the server device.

Even if it is described that the processes and the procedures described in the present description are executed by a single device, software, a component, or a module, such processes or procedures can be executed by a plurality of devices, a plurality of pieces of software, a plurality of components, and/or a plurality of modules. Further, even if it is described that various types of information described in the present description are stored in a single memory or storage unit, such information can be stored in a distributed manner in a plurality of memories included in a single device or the plurality of memories arranged in a distributed manner in a plurality of devices. Moreover, software and hardware elements described in the present description can be achieved by integrating the elements into fewer components or decomposing the elements into more components.

### Reference Signs List

- 1: Processing system
- 100: Processing device
- 200: Imaging device
- 201: Main body
- 202: Grip
- 203: Display
- 210: Input interface
- 211: Camera
- 212: Light source
- 212-1: Light source
- 213: Processor
- 214: Memory
- 215: Display panel
- 216: Communication interface
- 217: Engagement protrusion
- 218: Positioning protrusion
- 219: Diffusion plate
- 220: Capture button
- 221: Power button
- 222: Distal end
- 223: Accommodation space
- 224: Wall portion
- 225: Proximal end
- 226: Notifier
- 230: Connecting portion
- 231: Substrate
- 233: First polarizing plate
- 234: Second polarizing plate
- 300: Assistance tool
- 311: Gripping plate
- 312: Main body
- 313: Cover
- 314: Distal end
- 315: Tongue depressor
- 316: Proximal end
- 317: Piece
- 318: Engagement protrusion
- 319: Groove
- 320: Opening
- 321: Cover collar
- 322: Notification window
- 323: Through hole
- 325: Recess
- 326: Coating layer
- 327: Translucent resin layer
- 328: Connecting piece
- 329: Positioning mechanism
- 330: Insertion hole
- 331: Positioning piece
- 331a: Inclined surface
- 331b: Recess
- 332: Thin-walled area
- 333a: Notch
- 333b: Notch
- 400: Mounting table
- 600: User
- 700: Subject person

## Claims

1. An assistance tool to be inserted into an oral cavity for imaging an inner side of the oral cavity by an imaging device, the assistance tool comprising:
a main body in a cylindrical shape;
an opening being formed at an end portion of the main body in a direction opposite to a direction where the assistance tool is inserted into the oral cavity in a manner that the imaging device can be inserted into an interior of the main body;
a cover being formed at an end portion of the main body in the direction where the assistance tool is inserted into the oral cavity in a manner that light detected by the imaging device can pass through the cover; and
a cover collar being formed to connect the main body and the cover in a manner that the cover is disposed at the end portion of the main body in the direction where the assistance tool is inserted into the oral cavity, at least a part of the cover collar being included within an angle of view imaged by the imaging device inserted from the opening.

2. The assistance tool according to claim 1, wherein
the cover collar includes an appearing-in-image area formed in a manner that at least part thereof is included within the angle of view, and
the appearing-in-image area includes a structure formed in a predetermined pattern.

3. The assistance tool according to claim 1 or 2, further comprising a notification window for making a notifier visible in a case where the imaging device having the notifier for notifying that the imaging device is attached to the assistance tool is inserted from the opening.

4. The assistance tool according to claim 3, wherein
the notification window is disposed at a position facing the notifier in the case where the imaging device is inserted from the opening,
the notifier includes a light emitter that emits light to an outer side, and
at least a periphery of the light emission window inhibits transmission of at least a part of the light emitted from the light emitter.

5. The assistance tool according to any one of claims 1 to 4, further comprising a plate being disposed along at least a part of an outer periphery of the main body to protrude from the main body toward an outer peripheral direction of the main body in a direction perpendicular or substantially perpendicular to the direction where the assistance tool is inserted into the oral cavity.

6. The assistance tool according to claim 5, wherein the plate includes a through hole penetrating in a thickness direction.

7. The assistance tool according to any one of claims 1 to 6, further comprising a positioning mechanism for positioning the imaging device and the assistance tool in the case where the imaging device is inserted from the opening.

8. The assistance tool according to claim 7, wherein the positioning mechanism is connected to the main body with a connecting piece formed to be deformable or cuttable by applying a load in a predetermined direction.

9. The assistance tool according to claim 8, wherein the connecting piece is deformed or cut by applying the load to the connecting piece in the predetermined direction, and thus reinsertion of the imaging device into the assistance tool is inhibited.

10. An imaging device to be inserted into an interior of a main body of an assistance tool from an opening of the assistance tool, the assistance tool being inserted into an oral cavity for imaging an inner side of the oral cavity, and including: the main body having a shape; the opening being formed at an end portion of the main body in a direction opposite to a direction where the assistance tool is inserted into the oral cavity; a cover being formed at an end portion of the main body in the direction where the assistance tool is inserted into the oral cavity; and a cover collar being formed to connect the main body and the cover in a manner that the cover is disposed at the end portion of the main body in the direction where the assistance tool is inserted into the oral cavity, the imaging device comprising:
at least one processor, wherein
the at least one processor is configured to perform a process of
starting capturing a subject image by receiving an operation input by a user, and
based on the captured subject image, outputting information indicating that the imaging device is not inserted into the assistance tool in a case where at least a part of the cover collar is not included as a result of determining whether or not at least the part of the cover collar is included in the subject image.

11. The imaging device according to claim 10, wherein the determination is made by inputting the captured subject image to a learned assistance tool attachment determination model obtained by learning based on a subject image for learning classified according to whether or not at least a part of the cover collar is included in the subject image.

12. A program that causes an imaging device to be inserted into an interior of a main body of an assistance tool from an opening of the assistance tool to function as a processor configured to perform a process of
starting capturing a subject image by receiving an operation input by a user, and
based on the captured subject image, outputting information indicating that the imaging device is not inserted into an assistance tool in a case where at least a part of a cover collar is not included as a result of determining whether or not at least the part of the cover collar is included in the subject image, wherein
the assistance tool is inserted into an oral cavity for imaging an inner side of the oral cavity, and includes: the main body in a cylindrical shape; the opening being formed at an end portion of the main body in a direction opposite to a direction where the assistance tool is inserted into the oral cavity; a cover being formed at an end portion of the main body in the direction where the assistance tool is inserted into the oral cavity; and the cover collar being formed to connect the main body and the cover in a manner that the cover is disposed at the end portion of the main body in the direction where the assistance tool is inserted into the oral cavity.

13. A method that is executed by at least one processor included in an imaging device to be inserted into an interior of a main body of an assistance tool from the opening of an assistance tool, the assistance tool being inserted into an oral cavity for imaging an inner side of the oral cavity, and including: the main body in a cylindrical shape; the opening being formed at an end portion of the main body in a direction opposite to a direction where the assistance tool is inserted into the oral cavity; a cover being formed at an end portion of the main body in the direction where the assistance tool is inserted into the oral cavity; and a cover collar being formed to connect the main body and the cover in a manner that the cover is disposed at the end portion of the main body in the direction where the assistance tool is inserted into the oral cavity, the method comprising:
a stage of starting capturing a subject image by receiving an operation input by a user, and
a stage of outputting information indicating that the imaging device is not inserted into the assistance tool in a case where at least a part of the cover collar is not included as a result of determining whether or not at least the part of the cover collar is included in the subject image based on the captured subject image.
